# EUROPEAN PATENT APPLICATION

(11) **EP 4 360 532 A1**
(43) Date of publication of application: **01.05.2024**
(21) Application number: 22833654.1
(22) Date of filing: 30.06.2022
(51) Int. Cl.: A61B 1/00, A61B 1/04, A61B 34/00, H01F 7/06, H01F 7/18, H01F 3/10

(54) **MAGNETIC FIELD GENERATION MODULE AND MAGNETIC FIELD GENERATION DEVICE COMPRISING SAME**

(30) Priority: 02.07.2021 KR 20210086839
(71) Applicant: IUCF-HYU (INDUSTRY-UNIVERSITY COOPERATION FOUNDATION HANYANG UNIVERSITY), Seoul 04763 (KR)
(72) Inventor: JANG, Gunhee, Seoul 06326 (KR); LEE, Daehee, Seoul 04763 (KR); KIM, Seung Uk, Seoul 01309 (KR)
(74) Representative: Prüfer & Partner mbB Patentanwälte · Rechtsanwälte
(86) International application number: PCT/KR2022/009395
(87) International publication number: WO 2023/277600

(57) **Abstract**

A magnetic field generation module is disclosed. The magnetic field generation module comprises: a main core; at least two auxiliary cores positioned in the vicinity of and around the main core; auxiliary coils wound on respective auxiliary cores; and a power source unit for applying a current to the auxiliary coils.

## Description

### [Technical Field]

The present invention relates to a magnetic field generation module, and more particularly, to a magnetic field generation module capable of controlling a posture of a capsule endoscope.

### [Background Art]

A medical capsule endoscope has been developed to examine the digestive organs of patients. Because the existing capsule endoscope examines the stomach having a large space and has a difficulty in examining the lining of the stomach, it has been mainly used to examine the small intestine, in which it is difficult for a general endoscope to enter and the entire lining is allowed to be seen only through the peristalsis of the digestive organ.

However, in some cases, it may be difficult for the capsule endoscope to pass only by the peristalsis in a site in which the capsule endoscope passes from the stomach to the duodenum. In addition, because it is difficult to control a posture of the capsule endoscope in the desired direction, and accordingly, medical staff is required to individually check images taken from the capsule endoscope, time required for examination may be longer.

As the above description, there are limitations in examining all parts of the digestive organs, such as esophagus, stomach, duodenum, small intestine and large intestine, by using the capsule endoscopy. In order to overcome the above limitations, recently, several research institutes has been actively studied on attaching magnets to a capsule endoscope to control movements of the capsule endoscope using external magnetic fields.

### [Disclosure]

### [Technical Problem]

The present invention provides a magnetic field generation module and a magnetic field generation device including the same to control a posture of a capsule endoscope in order to examine all parts of the digestive organs such as esophagus, stomach, duodenum, small intestine and large intestine of a patient.

### [Technical Solution]

The magnetic field generation module according to the present invention includes: a main core; at least two auxiliary cores adjacent to the main core and arranged around the main core; auxiliary coils wound on the auxiliary cores, respectively; and a power source unit for applying a current to the auxiliary coils.

In addition, the auxiliary cores may be symmetrical about the main core.

In addition, the auxiliary cores may be arranged about the main core to have the same angle between adjacent auxiliary cores.

In addition, the main core may have a bottom with a curved surface convex downward or concave upward.

In addition, the auxiliary core may have a bottom with an inclined surface having one and opposite sides with different heights.

In addition, the magnetic field generation module may further include: a core support portion formed of a magnetic material to support the main core and the auxiliary cores.

In addition, the core support portion may include: a main support area for supporting the main core; and an auxiliary support area rotatable relative to the main support area to support the auxiliary core.

In addition, when the auxiliary support area is rotated, the auxiliary core may be moved between a first position in which a central axis of the auxiliary core is parallel to a central axis of the main core, and a second position in which the central axis of the auxiliary core is inclined with respect to the central axis of the main core.

In addition, the main core may have a diameter larger than a diameter of the auxiliary core.

In addition, the main core may be provided as a permanent magnet.

In addition, the magnetic field generation module may further include: a main coil wound around the main core and receiving a current from the power source unit; and a control unit for individually controlling the currents applied to the main coil and the auxiliary coils.

In addition, the control unit may apply a current to the main coil and block a current from being applied to the auxiliary coils.

In addition, the control unit may decrease a size of a current applied to any one of the auxiliary coils, and simultaneously increase a size of a current applied to another coil.

In addition, the control unit may control such that a size of the current applied to the main coil is different from a size of the current applied to the auxiliary coil.

The magnetic field generation device according to the present invention includes: a magnetic field generation module for generating a magnetic field; and a moving unit for moving the magnetic field generation module, wherein the magnetic field generation module may include: a main core; at least two auxiliary cores adjacent to and around the main core, in which the auxiliary coils are wound on the auxiliary cores; a core support portion formed of a magnetic material and connected to the moving unit to support the main core and the auxiliary cores; a power source unit for applying a current to the auxiliary coil; and a control unit for controlling the current applied to the auxiliary coil.

### [Advantageous Effects]

According to the present invention, the magnetic field generated from the magnetic field generation module is controlled, so that the capsule endoscope can have a posture controlled while being positioned perpendicular to an inner wall of the digestive organ, being inclined with respect to a central axis of the main core, or being rotated at an angle of 360 degrees around the central axis of the main core, and can photograph all parts of the digestive organ.

### [Description of Drawings]

FIG. 1 is a perspective view showing a magnetic field generation module according to the embodiment of the present invention.
FIG. 2 is a sectional view showing the magnetic field generation module in FIG. 1.
FIGS. 3 to 5 are views showing a posture control for a capsule endoscope using the magnetic field generation module of FIG. 1.
FIG. 6 is a view showing a magnetic field generation module according to another embodiment of the present invention.
FIG. 7 is a view showing a magnetic field generation module according to still another embodiment of the present invention.
FIG. 8 is a view showing a magnetic field generation module according to still another embodiment of the present invention.
FIGS. 9 and 10 are views showing a magnetic field generation module according to still another embodiment of the present invention.
FIG. 11 is a view showing a state in which a position of the capsule endoscope deviates from a main core due to a leakage magnetic field.
FIG. 12 is a view showing a magnetic field generation module according to still another embodiment of the present invention.
FIG. 13 is a view showing a magnetic field generation device according to the embodiment of the present invention.
FIG. 14 is a view showing a magnetic field generation device according to another embodiment of the present invention.

### [Best Mode]

The magnetic field generation module according to the present invention includes: a main core; at least two auxiliary cores adjacent to the main core and arranged around the main core; auxiliary coils wound on the auxiliary cores, respectively; and a power source unit for applying a current to the auxiliary coils.

### [Mode for Invention]

Hereinafter, exemplary embodiments of the present invention will be described in detail with reference to the accompanying drawings. However, the technical idea of the present invention is not limited to the exemplary embodiments described herein and may be embodied in other forms. Further, the embodiments are provided to enable contents disclosed herein to be thorough and complete and provided to enable those skilled in the art to fully understand the idea of the present invention.

Herein, when one component is mentioned as being on another component, it signifies that the one component may be placed directly on the other component or a third component may be interposed therebetween. In addition, in drawings, thicknesses of layers and areas may be exaggerated to effectively describe the technology of the present invention.

In addition, although terms such as first, second and third are used to describe various components in various embodiments of the present specification, the components will not be limited by the terms. The above terms are used merely to distinguish one component from another. Accordingly, a first component referred to in one embodiment may be referred to as a second component in another embodiment. Each embodiment described and illustrated herein may also include a complementary embodiment. In addition, the term "and/or" is used herein to include at least one of the components listed before and after the term.

The singular expression herein includes a plural expression unless the context clearly specifies otherwise. In addition, it will be understood that the term such as "include" or "have" herein is intended to designate the presence of feature, number, step, component, or a combination thereof recited in the specification, and does not preclude the possibility of the presence or addition of one or more other features, numbers, steps, components, or combinations thereof. In addition, the term "connection" is used herein to include both indirectly connecting a plurality of components and directly connecting the components.

In addition, in the following description of the embodiments of the present invention, the detailed description of known functions and configurations incorporated herein will be omitted when it possibly makes the subject matter of the present invention unclear unnecessarily.

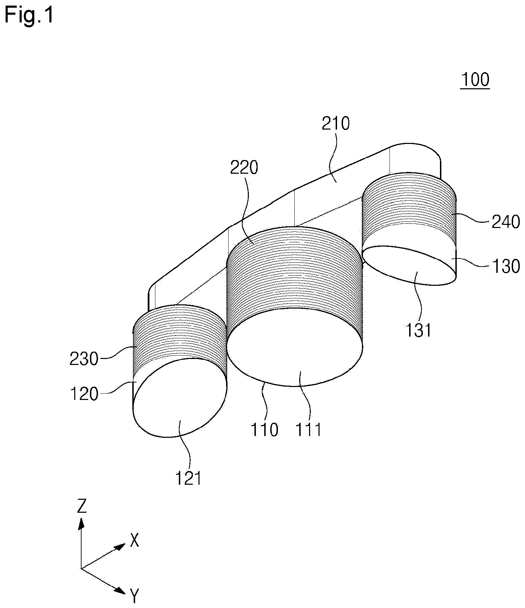

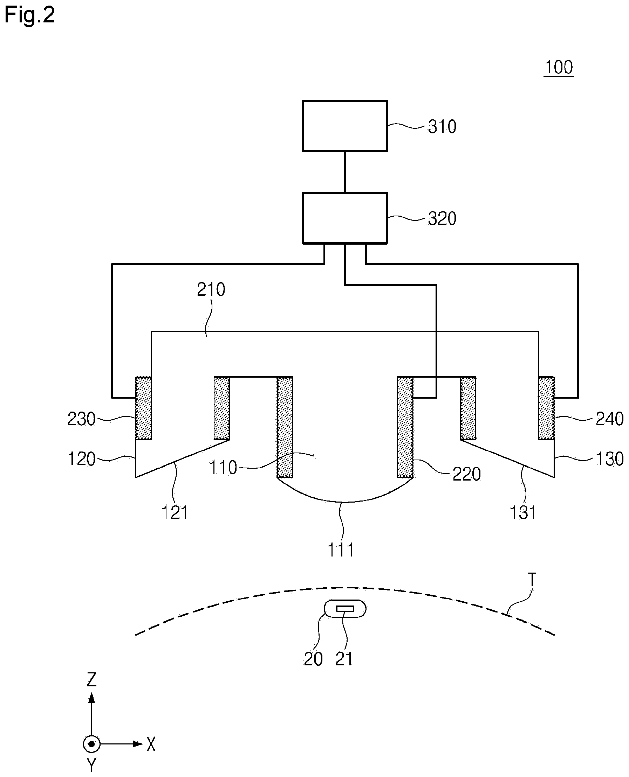

FIG. 1 is a perspective view showing a magnetic field generation module according to one embodiment of the present invention. FIG. 2 is a sectional view showing the magnetic field generation module in FIG. 1.

Referring to FIGS. 1 and 2, a magnetic field generation module 100 may generate and control a magnetic field and a magnetic force. The magnetic field and the magnetic force may be used to control the movement of a capsule endoscope 10 positioned within an operating area T. the capsule endoscope 20 is inserted into the human digestive organs and used to examine the digestive organs, and contains a permanent magnet 21 therein. Due to a magnetic torque formed by interaction between the magnetic field generated from the magnetic field generation module 100 and the permanent magnet of the capsule endoscope 20, the capsule endoscope 20 may have a posture controlled in the direction of the magnetic field.

A magnetic field generation module 100 includes a main core 110, auxiliary cores 120 and 130, a core support portion 210, a main coil 220, auxiliary coils 230 and 240, a power source unit 310, and a control unit 320.

The main core 110 has a predetermined shape, and is provided in a magnetic material. The main core 110 may have a cylindrical or polygonal pillar shape with a predetermined length. According to one embodiment, a bottom 111 of the main core 110 may be provided as a flat surface. According to another embodiment, the bottom of the main core 110 may have a central area protruding downward. The bottom of the main core 110 may have the central area provided as a downwardly convex curved surface. According to still another embodiment, the bottom of the main core 110 may have a central area indented upward. The bottom of the main core 110 may have the central area provided as a curved surface concavely indented upward. Due to the above shapes of the main core 110, magnetic flux density of the magnetic field generated from the main core 110 may be concentrated on the same line as a central axis of the main core 110.

The auxiliary core 120 and 130 has a predetermined shape, and is provided in a magnetic material. The auxiliary core 120 and 130 may have a cylindrical or polygonal pillar shape with a predetermined length. The auxiliary core 120 and 130 may have a diameter smaller than the main core 110.

At least two auxiliary core 120 and 130 are arranged adjacent to the main core 110 and around the main core 110. The number and angle of the arranged auxiliary core 120 and 130 may be variously modified. According to the embodiment, the arrangement of the auxiliary cores 120 and 130 may be symmetrical about the main core 110. In addition, angles between the auxiliary cores 120 and 130 adjacent to and about the main core 110 may be the same. When two auxiliary cores 120 and 130 are arranged, the auxiliary cores 120 and 130 may be arranged at an angle of 180 degrees on the same straight line with the main core 110 therebetween.

Bottoms 121 and 131 of the auxiliary cores 120 and 130 may be provided as a flat surface.

On the contrary, bottoms 121 and 131 of the auxiliary cores 120 and 130 may be provided as slopes with one side and the other side having different heights. The slopes may be toward the central axis of the main core 110 or may be directed outward.

According to one embodiment, as shown in FIG. 2, the bottoms 121 and 131 of the auxiliary cores 120 and 130 may be provided such that one side adjacent to the main core 110 is higher than the other side. Accordingly, the bottoms 121 and 131 of the auxiliary cores 120 and 130 are directed to the central axis of the main core 110. When a magnetic field is generated in the auxiliary cores 120 and 130, magnetic flux density of the magnetic field is concentrated on the other side of the bottom 121 and 131.

According to another embodiment, although not shown in the drawing, the bottoms 121 and 131 of the auxiliary cores 120 and 130 may be provided such that one side adjacent to the main core 110 is lower than the other side. Accordingly, bottoms 121 and 131 of the auxiliary cores 120 and 130 are directed outward. When a magnetic field is generated in the auxiliary cores 120 and 130, magnetic flux density of the magnetic field is concentrated on the one side of the bottom 121 and 131.

The core support portion 210 is formed of a magnetic material, and supports the main core 110 and the auxiliary cores 120 and 130. The core support portion 210 is connected to an upper end of the main core 110 and upper ends of the auxiliary cores 120 and 130. The magnetic field generated in the main core 110 and the auxiliary cores 120 and 130 is transmitted to the core support portion 210.

The main coil 220 is wound around the main core 110, and the auxiliary coils 230 and 240 are wound around the auxiliary cores 120 and 130, respectively. The number of wound times of the main coil 220 and the auxiliary coils 230 and 240 may be variously modified. According to embodiments, the number of wound times of the main coil 220 and the auxiliary coils 230 and 240 may be the same. On the contrary, the number of wound times of the main coil 220 and the auxiliary coils 230 and 240 may be different. In addition, the number of wound times of the auxiliary coils 230 and 240 around the auxiliary cores 120 and 130 may be the same or different.

The power source unit 310 applies currents to the main coil 220 and the auxiliary coils 230 and 240.

The control unit 320 individually controls the currents applied to the main coil 220 and the auxiliary coils 230 and 240. When the posture of the capsule endoscope 20 is controlled, the control unit 320 may control such that a current is always applied to the main coil 220. In addition, the control unit 320 may control such that a current is applied to the main coil 220, and a current is blocked from being applied to the auxiliary coils 230 and 240. In addition, the control unit 320 may control such that a size of the current applied to the main coil 220 is different from a size of the current applied to the auxiliary coils 230 and 240. In addition, the control unit 320 may control such that sizes of currents applied to the auxiliary coils 230 and 240 are different from each other. In addition, the control unit 320 may decrease a size of a current applied to any one of the auxiliary coils 230 and 240, and simultaneously increase a size of a current applied to another coil.

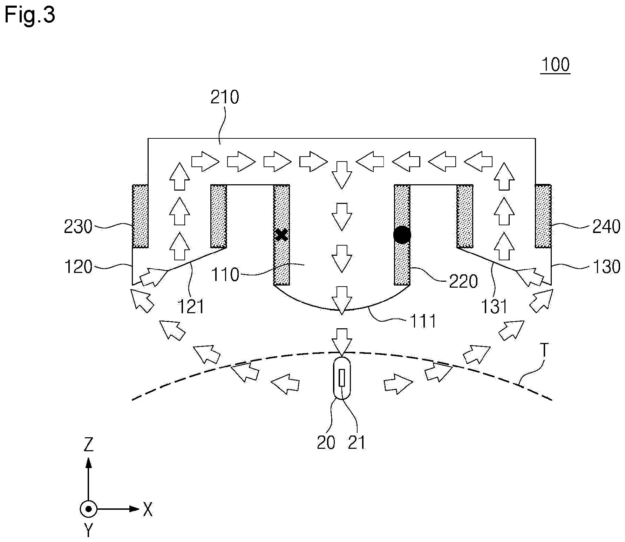

FIGS. 3 to 5 are views showing a posture control for a capsule endoscope using the magnetic field generation module of FIG. 1.

First, referring to FIG. 3, the control unit 310 controls such that a current is always applied to the main coil 220. In addition, controls such that a current is blocked from being applied to the auxiliary coils 230 and 240. When the current is applied to the main coil 220, a magnetic field and a magnetic force are formed around the main core 110 in a Z-axis direction. The magnetic flux density is concentrated on the same line as the central axis of the main core 110 in the central area of the bottom 111 of the main core 110. Accordingly, the capsule endoscope 20 may have a posture controlled in the Z-axis direction. Due to the posture control of the capsule endoscope 20, the capsule endoscope 20 may be fixed perpendicular to the stomach wall.

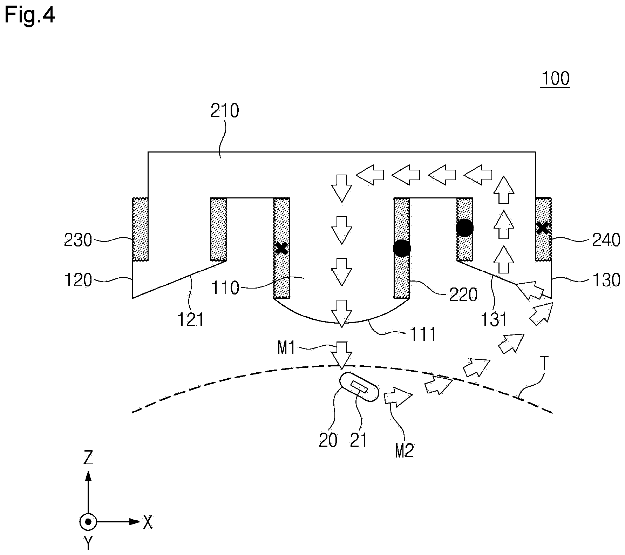

Referring to FIG. 4, the control unit 310 controls such that a current is applied to the main coil 220 and a current is simultaneously applied to one auxiliary coil 240. When the currents are applied, a magnetic field and a magnetic force are formed around the main core 220 and the auxiliary core 240. The magnetic flux density of the magnetic field formed around the auxiliary core 240 is concentrated on the other side of the bottom 241 of the auxiliary core 240. The magnetic field formed in the main core 110, the auxiliary core 130 and the core support portion 210 and the magnetic field formed in the operating area T disposed therein with the capsule endoscope 20 form a closed loop magnetic field. In the closed loop magnetic field, the capsule endoscope 20 may have a posture controlled at an oblique angle by a magnetic field M1 formed in the Z-axis direction and a magnetic field M2 formed inclined with respect to the Z-axis direction. Due to the posture control of the capsule endoscope 20, the capsule endoscope 20 may be fixed to be inclined with respect to the stomach wall. A size of the angle at which the capsule endoscope 20 is inclined may be adjusted by sizes of the currents applied to the main coil 220 and the auxiliary coil 240.

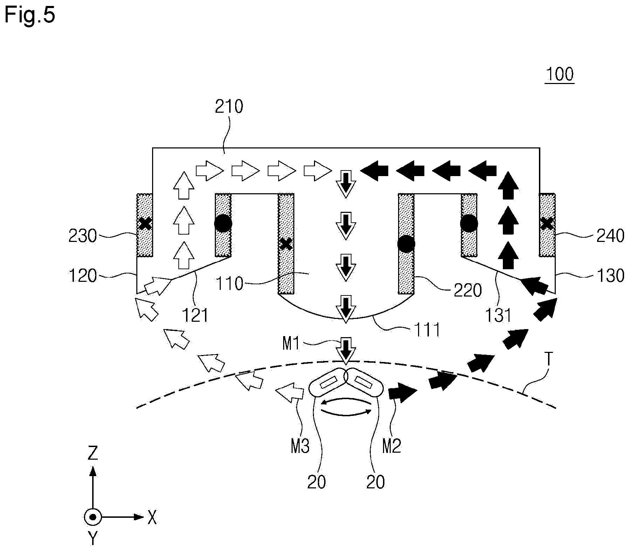

Referring to FIG. 5, while currents are applied to the main coil 220 and the one auxiliary coil 240, the control unit 320 decreases the size of the current applied to the auxiliary coil 230 and applies a current to the other auxiliary coil 230. In addition, the current applied to the auxiliary coil 230 is controlled to gradually become larger than the current applied to the auxiliary coil 240.

When the currents are applied to the main coil 220 and the auxiliary coil 240, a closed loop magnetic field is formed toward the auxiliary coil 240, and the capsule endoscope 20 may have a posture controlled at an oblique angle by the magnetic field M2 inclined with respect to the Z-axis direction.

In addition, when the sizes of the currents applied to the auxiliary coils 230 and 240 vary, a closed-loop magnetic field is formed toward the auxiliary coil 230 having the larger size of the applied current, and the capsule endoscope 20 may have a posture controlled at an oblique angle by the magnetic field M3 inclined with respect to the Z-axis direction.

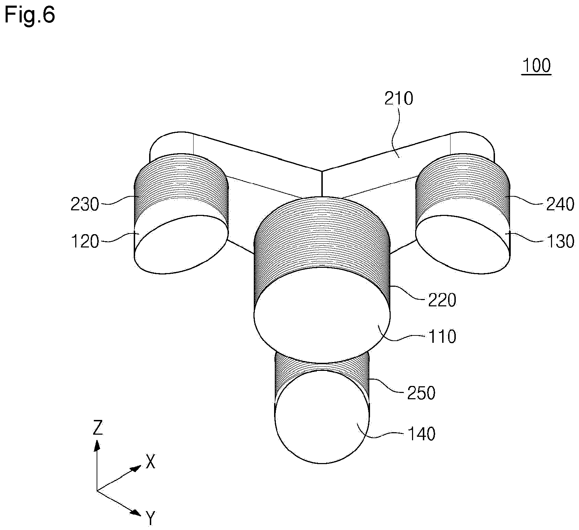

FIG. 6 is a view showing a magnetic field generation module according to another embodiment of the present invention.

Referring to FIG. 6, the magnetic field generation module 100 is configured such that three auxiliary cores 120, 130 and 140 are arranged around the main core 110. The auxiliary cores 120, 130 and 140 may be arranged at an angle of 120 degrees around the main core 110. The main coil 220 is wound around the main core 110, and the auxiliary coils are wound around the auxiliary cores 230, 240, and 250, respectively.

When a current is applied only to the main coil 220 under the control of the control unit 320, the capsule endoscope 20 may have a posture controlled in the Z-axis direction. In addition, when currents are applied to the main coil 220 and a first auxiliary coil 120, the capsule endoscope 20 may have a posture changed obliquely toward the first auxiliary coil 120.

In addition, while the currents are applied to the main coil 220 and the first auxiliary coil 230 under the control of the control unit 320, the size of the current applied to the first auxiliary coil 230 is decreased, a current is simultaneously applied to an adjacent second auxiliary coil 240, and a size of the applied current is gradually increased. Accordingly, the capsule endoscope 20 has a posture changed obliquely from the first auxiliary coil 230 toward the second auxiliary coil 240. In addition, the control unit 320 decreases the size of the current applied to the second auxiliary coil 240, simultaneously applies a current to a third auxiliary coil 250, and gradually increases a size of the applied current. Accordingly, the capsule endoscope 20 has a posture changed obliquely from the second auxiliary coil 240 toward the third auxiliary coil 250.

When the above-described control of the control unit 320 proceeds sequentially and continuously, the capsule endoscope 20 may have a posture sequentially changed at an angle of 120 degrees in the direction of the auxiliary coils 230, 240 and 250 to which a current is applied, so as to be rotated about the central axis of the main core 110.

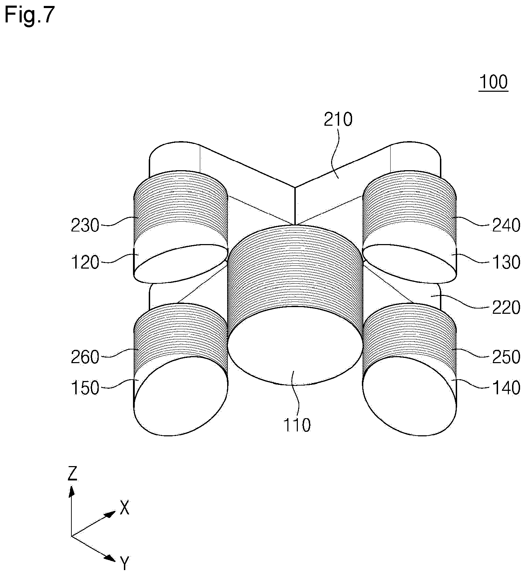

FIG. 7 is a view showing a magnetic field generation module according to still another embodiment of the present invention.

Referring to FIG. 7, a magnetic field generation module 100 is configured such that four auxiliary cores 120 to 150 are arranged around the main core 110. The auxiliary cores 120 to 150 may be arranged at an angle of 90 degrees around the main core 110. The main coil 220 is wound around the main core 110, and the auxiliary coils 230 to 250 are wound around the auxiliary cores 120 to 150, respectively.

Under the control of the control unit 320, a current is sequentially and continuously applied to the first to fourth auxiliary coils 230 to 250. When a size of the applied current is adjusted, the capsule endoscope 20 may have a posture changed toward the auxiliary coils 230 to 250 in which the applied current has a larger size, and thus may be rotated at an angle of 90 degrees about the central axis of the main core 110.

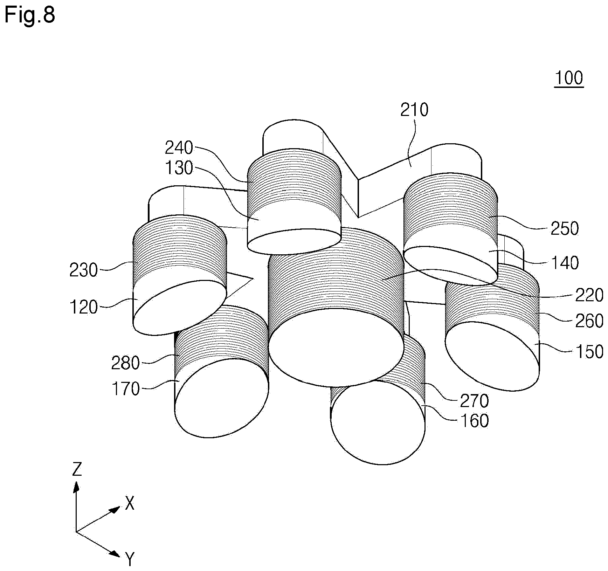

FIG. 8 is a view showing a magnetic field generation module according to still another embodiment of the present invention.

Referring to FIG. 8, the magnetic field generation module 100 is configured such that six auxiliary cores 120 to 170 are arranged around the main core 110. The auxiliary cores 120 to 170 may be arranged at an angle of 60 degrees around the main core 110. The main coil 220 is wound around the main core 110, and the auxiliary coils 230 to 280 are wound around the auxiliary cores 120 to 170, respectively.

Under the control of the control unit 320, a current is sequentially and continuously applied to the first to sixth auxiliary coils 230 to 280. When a size of the applied current is adjusted, the capsule endoscope 20 may have a posture changed toward the auxiliary coils 230 to 280 in which the applied current has a larger size, and thus may be rotated at an angle of 60 degrees about the central axis of the main core 110.

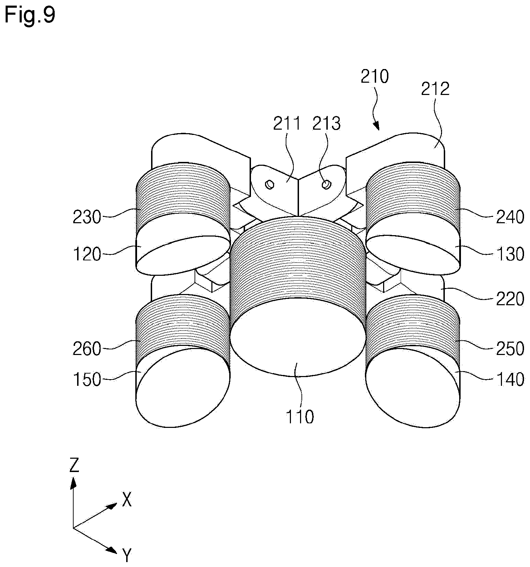

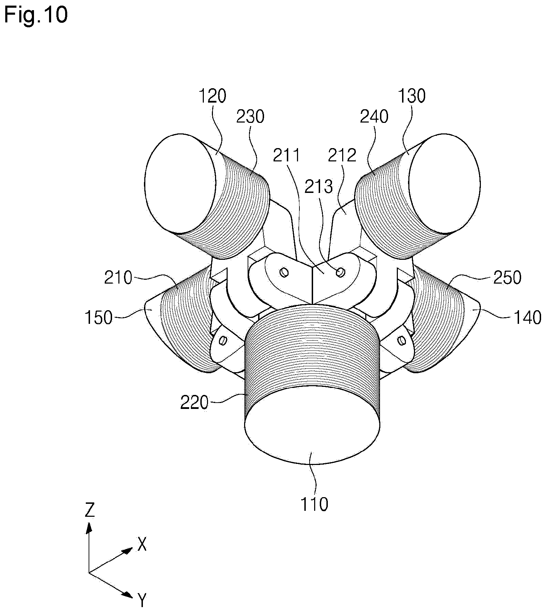

FIGS. 9 and 10 are views showing a magnetic field generation module according to still another embodiment of the present invention. FIG. 9 shows a state before a second area of the core support portion is rotated. FIG. 10 is a view showing a state after the second area of the core support portion is rotated.

Referring to FIGS. 9 and 10, the core support portion 210 of the magnetic field generation module 100 includes a first area 211 and a second area 212. The first area 211 is positioned in a central area of the core support portion 210 and supports the main core 220.

The second area 212 is provided in a number corresponding to the auxiliary cores 120 to 150 and supports the auxiliary cores 120 to 150. The second area 212 is provided radially around the first area 211. The second area 212 is coupled to the first area 211 through an axis 213 so as to be relatively rotated with respect to the first area 211. The second area 212 may be rotated in the Z-axis direction around the axis 213 perpendicular to the longitudinal direction thereof.

When the second area 212 is rotated, the auxiliary cores 120 to 150 may be moved between a first position in which a central axis of the auxiliary core is parallel to a central axis of the main core 110 (see FIG. 9), and a second position in which the central axis of the auxiliary core is inclined with respect to the central axis of the main core 110 (see FIG 10) .

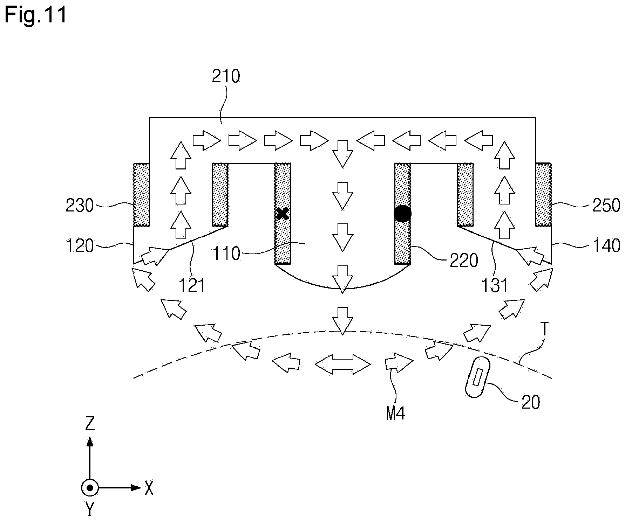

When the posture of the capsule endoscope 20 is vertically controlled while the auxiliary cores 120 to 150 are disposed in the first position, a current is applied only to the main coil 220, and a current is blocked from being applied to the auxiliary coils 230 to 260. At this time, as shown in FIG. 11, the capsule endoscope 20 may be positioned close to the auxiliary core 140 depending on relative positions between the main core 110 and the capsule endoscope 20 due to a leakage magnetic field M4 generated from the main coil 220 toward the auxiliary coils 230 to 260.

In order to solve the above problem, the second area 212 is rotated to position the auxiliary cores 120 to 150 in the second position as shown in FIG. 10. In this case, since the leakage magnetic field from the main coil 220 toward the auxiliary coils 230 to 260 is reduced, the posture of the capsule endoscope 20 may be stably fixed in the Z-axis direction below the main core 110.

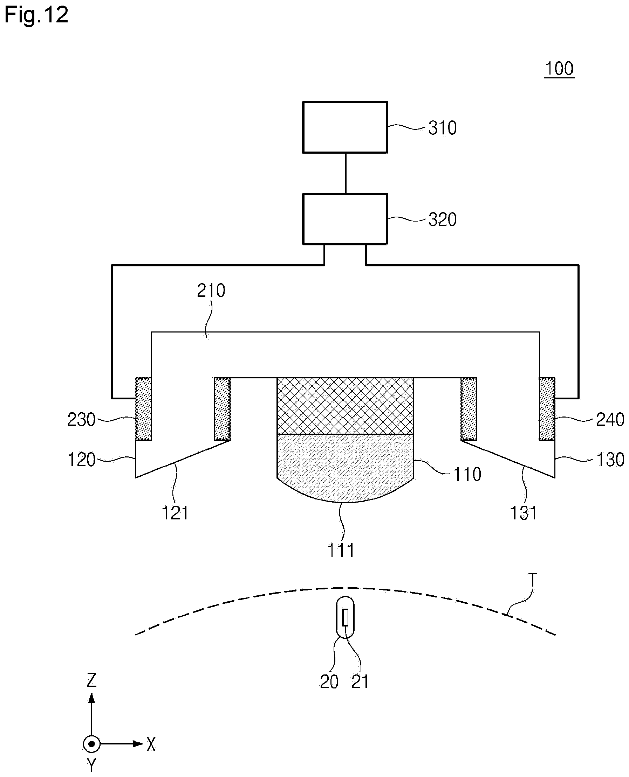

FIG. 12 is a view showing a magnetic field generation module according to still another embodiment of the present invention.

Referring to FIG. 12, the magnetic field generation module 100 includes a main core 110, auxiliary cores 120 and 130, core support portion 210, auxiliary coils 230 and 240, a power source unit 310, and a control unit 320.

The main core 110 may be provided to have the same shape as described in FIGS. 1 and 2. The main core 110 may be provided as a permanent magnet. The main core 110 may have an N pole and an S pole aligned in the Z-axis direction. When the main core 110 is provided as a permanent magnet, a magnetic field is always formed around the main core 110. As described with reference to FIGS. 1 to 11, this has the same effect as a magnetic field formed around the main core 110 by applying a current to the main coil 220. Due to the magnetic field generated in the main core 110, the capsule endoscope 20 may have a posture controlled in the Z-axis direction.

The auxiliary cores 120 and 130, the core support portion 210, and the auxiliary coils 230 and 240 may be provided as any one of the embodiments described in FIGS. 1 to 11, and accordingly, detailed description will be omitted.

The power source unit 310 applies a current to the auxiliary coils 230 and 240.

The control unit 320 controls the current applied to the auxiliary coils 230 and 240. According to the embodiment, when the capsule endoscope 20 is controlled to have a posture arranged in the Z-axis direction, the control unit 320 blocks the current applied to the auxiliary coils 230 and 240. In addition, when the posture of the capsule endoscope 20 is controlled at an oblique angle relative to the Z-axis direction, the control unit 320 may apply a current to one auxiliary coil 240 and block a current from being applied to the other auxiliary coil 230. On the contrary, the control unit 320 may apply a current to one auxiliary coil 240 in which the current is larger than a current applied to the other auxiliary coil 230. In addition, when the capsule endoscope 20 is rotated about the central axis of the main core 110, the control unit 320 may decrease a size of a current applied to any one of the auxiliary coils 230 and 240, and simultaneously increase a size of a current applied to another coil. When the current application is sequentially and continuously controlled, the capsule endoscope 20 may be rotated in units of angles between the auxiliary cores 230 and 240.

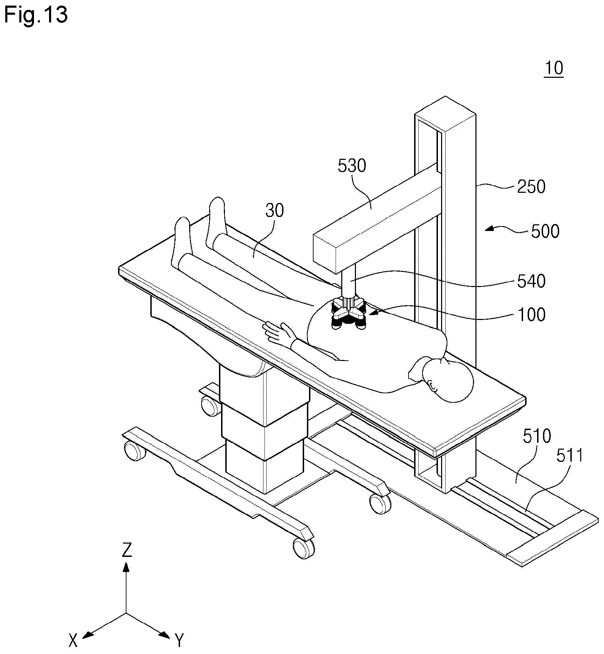

FIG. 13 is a view showing a magnetic field generation device according to the embodiments of the present invention.

Referring to FIG. 13, the magnetic field generation device 10 includes a magnetic field generation module 100 and a moving unit 500. Any one of the magnetic field generation modules 100 described in FIGS. 1 to 12 may be used for the magnetic field generation module 100.

The moving unit 500 moves the magnetic field generation module 100. Specifically, the moving unit 500 positions the magnetic field generation module 100 above the abdomen of a patient 30 having swallowed the capsule endoscope 20, and linearly moves the magnetic field generation module 100 in the X-axis, Y-axis and Z-axis directions.

The moving unit 500 includes a support plate 510, a support shaft 520, a moving arm 530 and a support rod 540.

The support plate 510 is installed on the ground, and has a longitudinal direction provided in the Y-axis direction. A pair of first guide rails 511 are arranged parallel to each other in the Y-axis direction on a top surface of the support plate 510.

The support shaft 520 is provided to have a predetermined length, and a longitudinal direction thereof is provided in the Z-axis direction. The support shaft 520 is installed on the support plate 510, and movable in the Y-axis direction along the first guide rail 511. The support shaft 520 has one opened side surface and is provided therein with , a second guide rail (not shown) in the Z-axis direction.

The moving arm 530 is provided to have a predetermined length, and has a longitudinal direction arranged in the X-axis direction. The moving arm 530 has one end inserted into the support shaft 520 and coupled to the second guide rail. The moving arm 530 may be moved in the Z-axis direction along the second guide rail.

The support rod 540 is coupled to the other end of the moving arm 530, and provided as a structure having an adjustable length. The support rod 540 has a longitudinal direction arranged in the Z-axis direction, and has a lower end to which the magnetic field generation module 100 is coupled. When the length of the support rod 540 is adjusted, the magnetic field generation module 100 may be moved in the Z-axis direction.

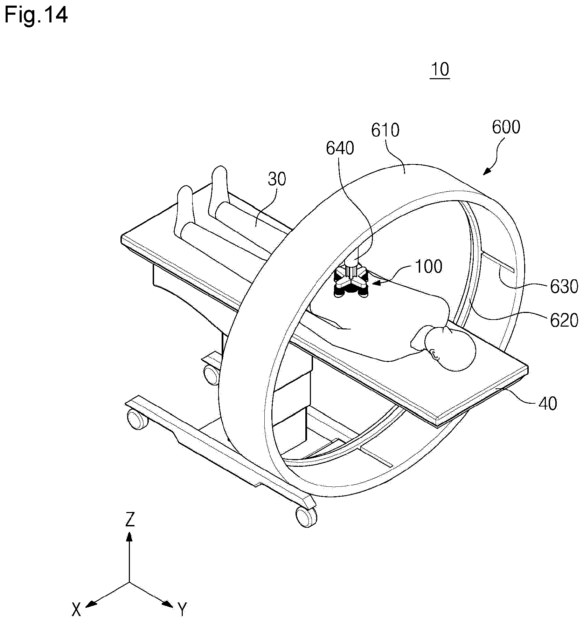

FIG. 14 is a view showing a magnetic field generation device according to another embodiment of the present invention.

Referring to FIG. 14, the moving unit 600 includes a support body 610, a first guide rail 620, a second guide rail 630, and a support rod 640.

The support body 610 is a ring-shaped structure having a predetermined diameter and width, and a bed 40 on which the patient 30 lies is positioned therein.

The first guide rail 620 has a ring shape having a diameter corresponding to the support body 610, and is fixedly installed on an inner side of the support body 610. The first guide rail 620 is installed adjacent to a rear end of the support body 610.

The second guide rail 630 has a length corresponding to a width of the support body 610 in the Y-axis direction and is provided in plural numbers. The second guide rails 630 are spaced apart at predetermined intervals along a circumference of the inner side of the support body 610. The second guide rail 630 has one end connected to the first guide rail 620.

The support rod 640 is provided in a predetermined length, and is provided as a structure having an adjustable length. The magnetic field generation module 100 is coupled to an end of the support rod 640. The support rod 640 may be moved along the first guide rail 620 and the second guide rail 630. Specifically, the support rod 640 may be rotated around the patient 30 along the first guide rail 620, and linearly moved in the Y-axis direction along the second guide rail 630. In addition, a distance between the magnetic field generation module 100 and the patient 30 may be adjusted by adjusting the length of the support rod 630.

When the above-described moving units 500 and 600 are driven, the magnetic field generation module 100 may control a posture of the capsule endoscope 20 in all parts of the patient's digestive organs, such as the esophagus, stomach, duodenum, small intestine and large intestine. In addition, the posture of the capsule endoscope 20 is controlled, so that all parts of the patient's digestive organs can be examined.

Although the present invention has been described in detail with reference to the preferred embodiments, the present invention is not limited to the specific embodiments and shall be interpreted by the following claims. In addition, it will be apparent that a person having ordinary skill in the art may carry out various deformations and modifications for the embodiments described as above within the scope without departing from the present invention.

### [Industrial Applicability]

A magnetic field generation module and a magnetic field generation device including the same according to the embodiments of the present invention may be used to examine all parts of the digestive organs such as esophagus, stomach, duodenum, small intestine and large intestine of a patient by controlling a posture of a capsule endoscope.

## Claims

1. A magnetic field generation module comprising:
a main core;
at least two auxiliary cores adjacent to the main core and arranged around the main core;
auxiliary coils wound on the auxiliary cores, respectively; and
a power source unit for applying a current to the auxiliary coils.

2. The magnetic field generation module of claim 1, wherein the auxiliary cores are symmetrical about the main core.

3. The magnetic field generation module of claim 1, wherein the auxiliary cores are arranged about the main core to have a same angle between adjacent auxiliary cores.

4. The magnetic field generation module of claim 1, wherein the main core has a bottom with a curved surface convex downward or concave upward.

5. The magnetic field generation module of claim 1, wherein the auxiliary core has a bottom with an inclined surface having one and opposite sides with different heights.

6. The magnetic field generation module of claim 1, further comprising:
a core support portion formed of a magnetic material to support the main core and the auxiliary cores.

7. The magnetic field generation module of claim 6, wherein the core support portion includes:
a main support area for supporting the main core; and
an auxiliary support area rotatable relative to the main support area to support the auxiliary core.

8. The magnetic field generation module of claim 7, wherein, when the auxiliary support area is rotated, the auxiliary core is movable between a first position in which a central axis of the auxiliary core is parallel to a central axis of the main core, and a second position in which the central axis of the auxiliary core is inclined with respect to the central axis of the main core.

9. The magnetic field generation module of claim 1, wherein the main core has a diameter larger than a diameter of the auxiliary core.

10. The magnetic field generation module of claim 1, wherein the main core is provided as a permanent magnet.

11. The magnetic field generation module of claim 1, further comprising:
a main coil wound around the main core and receiving a current from the power source unit; and
a control unit for individually controlling the currents applied to the main coil and the auxiliary coils.

12. The magnetic field generation module of claim 11, wherein the control unit applies a current to the main coil, and blocks a current from being applied to the auxiliary coils.

13. The magnetic field generation module of claim 11, wherein the control unit decreases a size of a current applied to any one of the auxiliary coils, and simultaneously increases a size of a current applied to another coil.

14. The magnetic field generation module of claim 11, wherein the control unit controls such that a size of the current applied to the main coil is different from a size of the current applied to the auxiliary coil.

15. A magnetic field generation device comprising:
a magnetic field generation module for generating a magnetic field; and
a moving unit for moving the magnetic field generation module, wherein
the magnetic field generation module includes:
a main core;
at least two auxiliary cores adjacent to and around the main core, in which the auxiliary coils are wound on the auxiliary cores;
a core support portion formed of a magnetic material and connected to the moving unit to support the main core and the auxiliary cores;
a power source unit for applying a current to the auxiliary coil; and
a control unit for controlling the current applied to the auxiliary coil.
